# EUROPEAN PATENT SPECIFICATION

(11) **EP 3 429 642 B1**
(45) Date of publication and mention of the grant of the patent: **14.12.2022**
(21) Application number: 17718164.1
(22) Date of filing: 14.03.2017
(51) Int. Cl.: A61L 11/00, B09B 3/00, B02C 23/10, A61L 2/20

(54) **BIOMEDICAL WASTE TREATMENT ASSEMBLY AND METHOD**
ANLAGE UND VERFAHREN ZUR BEHANDLUNG VON BIOMEDIZINISCHEM ABFALL
ENSEMBLE ET PROCEDE DE TRAITEMENT DE DECHETS BIOMEDICAUX

(30) Priority: 14.03.2016 US 201662308165 P
(43) Date of publication of application: 23.01.2019
(73) Proprietor: Jude, Peter, Maple Lake, Minnesota 55359 (US); Pelegrene, Stephen, Plymouth, Minnesota 55442 (US); Hofer, Michael, Fargo, North Dakota 58104 (US)
(72) Inventor: Jude, Peter, Maple Lake, Minnesota 55359 (US); Pelegrene, Stephen, Plymouth, Minnesota 55442 (US); Hofer, Michael, Fargo, North Dakota 58104 (US)
(74) Representative: Schulz Junghans Patentanwälte PartGmbB
(86) International application number: PCT/US2017/022381
(87) International publication number: WO 2017/160891

(56) References cited:
- WO-A2-2009/019570
- WO-A2-2009/064538
- JP-A- H08 229 425
- KR-A- 20130 033 885
- US-A1- 2007 029 418
- US-A1- 2007 196 232
- US-A1- 2011 290 919
- US-A1- 2012 060 705
- US-B1- 8 518 339

## Description

Biomedical waste includes blood and blood products, tissues removed during surgery and disposable items used and discarded in the normal course of surgical and other medical procedures. Many cities and states mandate special processing of biomedical waste, typically by incineration, autoclaving, or chemical sterilization, to destroy disease-causing pathogens before allowing the waste into local landfills. However, incineration and autoclaving are expensive in terms of their energy usage and affect on local air quality, and chemical sterilizers are typically toxic, posing additional risks to waste-management workers and the environment.

In response to these shortcomings, some waste-treatment processors have built systems that sterilize waste using ozone gas, a form of oxygen that reverts to natural oxygen after performing its sterilization function. For example, U.S. Patent 7,550,111 describes a vertically oriented machine that drops biomedical waste from a top-mounted hopper through a shredder into an ozone-treatment chamber. The chamber holds and exposes the shredded waste to ozone for a period of time, such as 15 minutes, before conveying into a collection bin for later transport to a landfill.

One problem that the present inventors recognized with this system is that its waste-processing speed (throughput) is less than desirable for some medical facilities. The low throughput can result in prolonged storage of unsterilized waste as it awaits processing, exposing staff and patients not only to unpleasant odors, but also to a risk of infection.

In response to the throughput problem, the present inventors developed and patented the ozone-based system described in U.S. Patents 8,518,339; 8,652,405; and 8,784,746. This system, sold under the Clean Waste Systems brand, provided a significant boost in throughput over that described under 7,550,111 patent. However, not content to rest on their laurels, the present inventors recognized a further need for systems not only with greater throughput but also a smaller footprint to accommodate onsite processing of waste in smaller hospitals, clinics, and other medical facilities.

### Summary

The present invention is directed to a biomedical waste treatment assembly using ozone according to the set of claims.

One exemplary system includes a hopper, a shredder, a pressurized auger, and a waste receptacle, with hopper and shredder position to feed shredded waste into the auger. The pressurized auger is inclined and includes ozone injectors to treat waste as the auger conveys it upward toward an outlet that drops the sterilized waste into the waste receptacle.

In some embodiments, the pressurized auger exists within a first chamber having a first cross-sectional area and outputs waste through a port into a second chamber, with the port having a second cross-sectional area less than the first cross-sectional area, for example 50% or more less than that of the first chamber. The second chamber also has a length substantially less than that of the first chamber, and includes a mechanism for biasing shredded within the second chamber waste toward the first chamber, effectively creating a plug of shredded waste against the port to facilitate maintaining pressure and ozone concentration within the first chamber as waste is conveyed into the second chamber. In some embodiments, the bias mechanism includes a weighted door that pivots open in response to shredded waste moved by the auger through the port and against the door. Weighted plates can be added to or removed from the door in some embodiments to allow calibration of the plug seal. Still other embodiments provide an automatic tensioning or resistance mechanism on the door to dynamically adjust the plug seal to maintain predetermined pressure conditions within the first chamber or ozone levels within the second chamber.

### Brief Description of the Drawings

Various embodiments are described herein with reference to the following attached figures (Figs). These figures are annotated with reference numbers for various features and components, and these numbers are used in the following description as a teaching aid, with like numbers referring to the same or similar features and components.
Fig. 1 is a schematic diagram of an exemplary waste treatment system 100 which corresponds to one or more embodiments of the present invention.

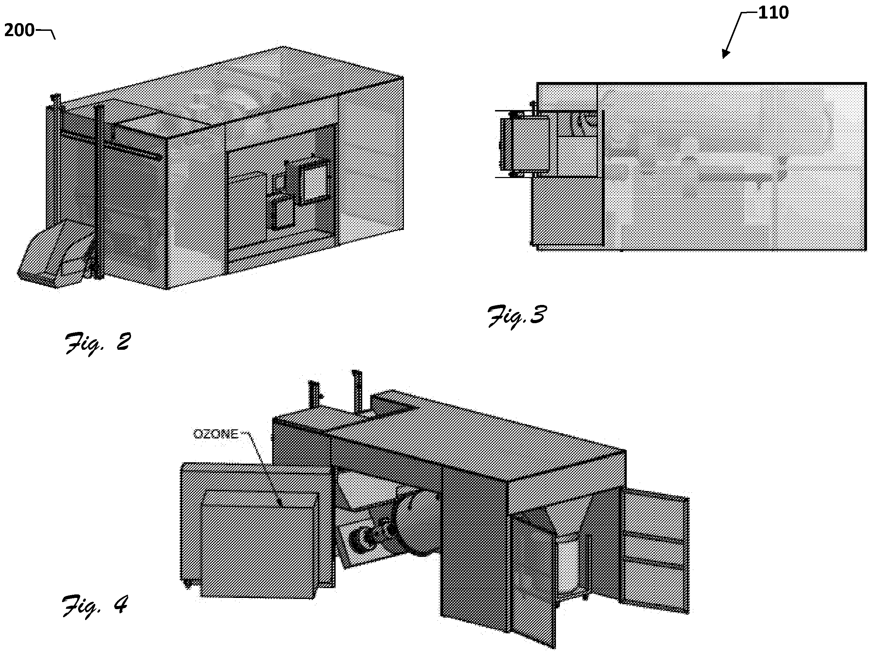

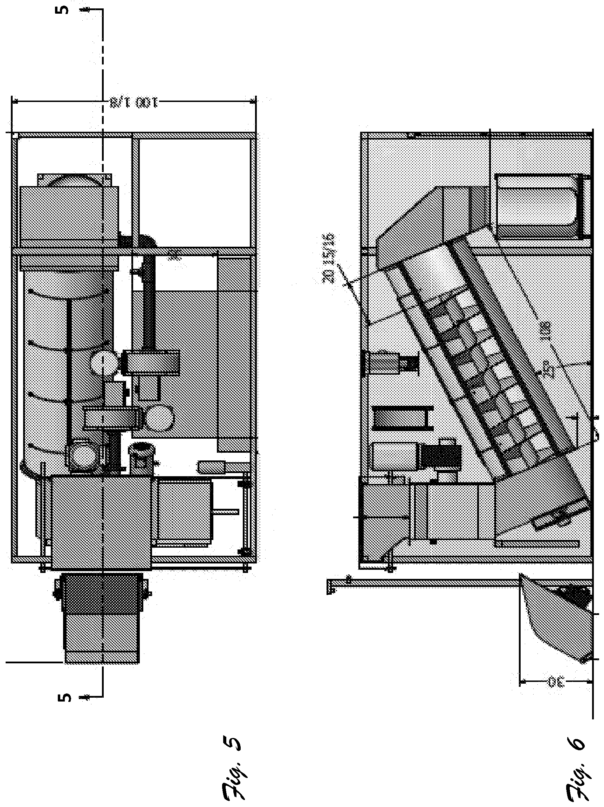

Figs. 2, 3, 4, 5, and 6 are views of an exemplary waste treatment system 200, which is similar in some respects to system 100 and which corresponds to one or more embodiments of the present invention.
FIG. 7 is a flow chart of an exemplary method of operating a waste treatment system, such as system 100 and/or system 200, and which corresponds to one or more embodiments of the present invention.

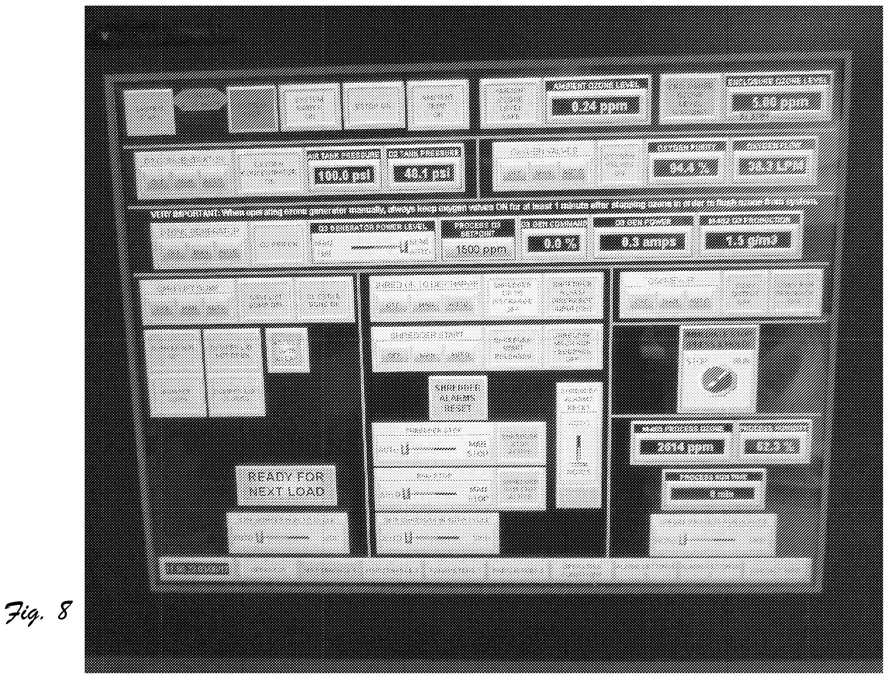

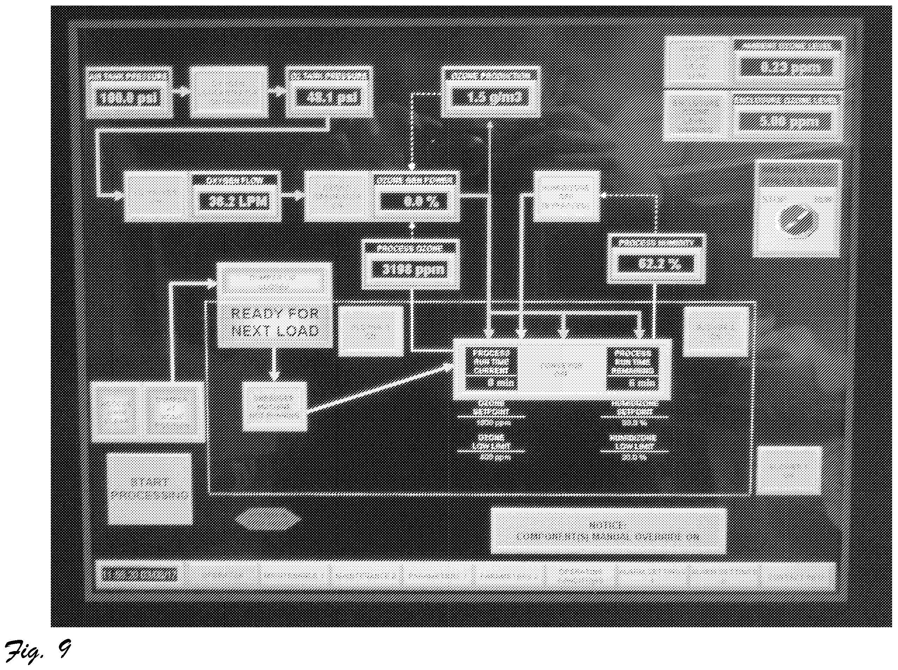

Figs. 8 and 9 are screen shots of a touch screen control interface display for use with system 100 and 300, and thus corresponding to one or more embodiments of the present invention.

### Detailed Description of Exemplary Embodiments

This document, which includes the referenced drawings and appended claims, describes one or more specific embodiments of one or more inventions. These embodiments, offered not to limit but only to exemplify and teach the invention(s), are shown and described in sufficient detail to enable those skilled in the art to implement or practice the inventions. Thus, where appropriate to avoid obscuring the invention, the description may omit certain information known to those of skill in the art.

This document may use relational terms, such as second, top and bottom, and the like. These terms may be used solely to distinguish one entity or action from another entity or action without necessarily requiring or implying any actual such relationship or order between such entities or actions. The terms "comprises," "comprising," "has", "having," "includes", "including," "contains", "containing" or any other variation thereof, are intended to be open ended, such that a process, method, article, or apparatus that comprises, has, includes, contains a list of elements does not include only those elements but may include other elements not expressly listed or inherent to such process, method, article, or apparatus. An element proceeded by "comprises a", "has ...a", "includes ...a", "contains ...a" does not, without further constraints, preclude the existence of additional identical elements in the process, method, article, or apparatus that comprises, has, includes, contains the element. The terms "a" and "an" are defined as one or more unless explicitly stated otherwise herein. The terms "substantially", "essentially", "approximately", "about" or any other version thereof, are defined as being close to as understood by one of ordinary skill in the art, and in one non-limiting embodiment the term is defined to be within 10%, in another embodiment within 5%, in another embodiment within 1% and in another embodiment within 0.5%. The term "coupled" as used herein is defined as connected, although not necessarily directly and not necessarily mechanically. A device or structure that is "configured" in a certain way is configured in at least that way, but may also be configured in ways that are not listed.

### Exemplary Systems

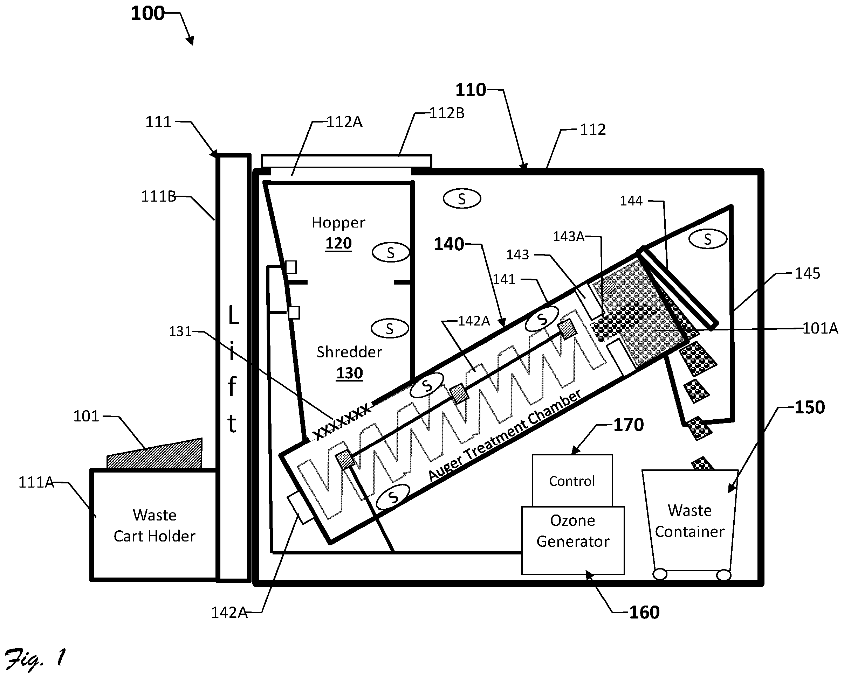

Fig. 1 shows an exemplary medical waste treatment system 100. System 100 includes a housing assembly 110, a hopper 120, a shredder 130, an inclined waste treatment chamber 140, a waste receptacle (or container) 150, a control module 170, and an ozone module 160.

Housing assembly110, formed of high grade plastic, steel, or other durable materials, includes a lift assembly 111 and a body 112. Lift assembly 111 includes a cart holder 111A and lift mechanism 111B. Cart holder 111A holds at least one cart of biomedical and/or pharmaceutical waste 101. In some embodiments, cart holder 111A also includes a digital scale or other waste analysis sensors not shown. Cart holder 111B is mechanically attached or linked to lift mechanism 111B, which may be electromechanical, hydraulic, or pneumatic and which is configured to lift waste cart holder 11A above the height of housing assembly 110 and dump its contents through opening 112A of body 112 into hopper 120.

Some embodiments include a lid assembly 112B which is opened and closed automatically in unison with or at a faster rate than the lifting and lower of the cart holder.)

Hopper 120 receives biomedical waste for processing. In the exemplar embodiment, the hopper has a capacity of 94.15 cubic meter (33.25 cubic feet) of material. In some embodiments, hopper 120 includes one or more ozone injectors for reducing waste odors and sainting the hopper. Moreover, some also embodiments also fluidly couple the hopper to an exhaust fan and an ozone destruct system for exhausting substantially ozone freewaste gases to atmosphere. Hopper 120 includes a bottom opening 121 which allows waste to fall under the weight of gravity onto shredder 130.

Shredder 130, which is positioned underneath hopper 110, receives waste from hopper 110. In the exemplary embodiment, shredder 120 includes a 2- or 4-shaft shredder and a lower opening covered by a screen 131. Exemplary shredders include SSI Shredder Systems Model Q55ED Shredder or Q70ED (36"x31" or 44"x40" cutting chamber) to shred the material by up to 90%. The exemplary screen includes openings of approximately 1.91-5.08 centimeters (0.75-2.0 inches) in diameter or maximal width for non-circular geometries which restricts the maximal size of the waste particles allowed to enter inclined waste treatment chamber 140.

Chamber 140 includes a body 141, a waste transporter or conveyor assembly 142, and a partition 143, an exit control member 144, waste exit chute 145, and ozone injectors 146. Body 141, formed for example of a substantially fluid-tight cylindrical metal, plastic (PVC), or composite tube, is generally inclined, for example at 20, 25, 30, 40, or 45, 50, 60, 70, 80 or in some instances even 90 degrees relative to horizontal, . The chamber which is also sealed from the remaining interior volume of housing body 112, contains waste transporter assembly 142.

Waste Transporter assembly 142, in the exemplary embodiment, takes the form of a screw type auger compactor, which includes an auger motor 142A and an auger screw 142B. The auger screw extends from a lower end of the chamber up to partition 143, which divides the chamber into a treatment portion 141A and an exit or plug portion 141B. Partition 143, formed or plastic or metal plate, includes a central circular port or opening, which is less than 75% of the diameter or maximal width of chamber body 141. In some embodiments, the port has a diameter that is 50% or less than that of the chamber. In still others, two, three, four, or five ports are provided. The function of the ports is allow portions of treated waste to pass from treatment portion 141A into exit portion 141B as auger screw 142B moves the waste in a helical pattern into the plane defined by the partition. Although the partition is shown a simple planar structure in the exemplary embodiment, some embodiments may form the partition as a parabolic, spherical, conical surface; and still others provide concave or even convex surface geometries . Some embodiments may combine the shredder and auger into a single machine. Also some embodiments use a Sani-Tech System ST1546/36-15HP Refuse & Recycling Compactor. The exit portion 141B is generally less than 20 or 30% of the full length of chamber 141, is terminated with an exit control member 143.

Exit control member 144 not only partly governs the rate of exit of treated waste from chamber 141, but also forces the treated waste within the exit portion to form a waste plug that at least partially seals off or constricts flow of ozone out of the chamber, thereby assisting in maintaining the desired ozone concentration and other sterilization conditions within the chamber. In the exemplary embodiment, the chamber takes the form of horizontally hinged metal door with optional weights to change the amount of back pressure the door applies to treated waste within the exit portion of the chamber, thereby setting the level of sealing of the chamber or density of treated waste within the exit portion. Some embodiments replace or augment this simple weighted mechanism with an automatic mechanical, electromechanical, hydraulic, or pneumatic arrangement that varies the back pressure of the door on the treated waste plug 101A to maintain a desired waste exit rate, waste density, chamber ozone concentration, or ozone leakage. In some embodiments, the door may include a raised interior portion that engages with the interior rim of the chamber to further restrict exit of waste around regions of the door closer to the hinge.

Waste exit chute 145 guides waste that falls past exit control member 144 into waste container 150. In some embodiment, waste container 150 includes wheels. In still others it includes automatic bagging equipment.

System 100 also includes ozone assembly 160 and control assembly 170. Ozone assembly 160 includes an ozone generator 161 and ozone distribution system 162. Ozone distribution system 162 includes ozone injectors 163A (in hopper), 163B (in shredder), 163C-E (in chamber) in fluid communication with ozone generator 161. Control assembly 170, which a controller 171, a touch-screen control panel 172, and a network or distributed sensor units, denoted S in the figure. Controller 171 includes one or more processing or control circuits, which provide control signals to various valves and safety interlocks (not shown) in accord with machine-readable and executable instructions and control parameters stored in memory. For example, in the exemplary embodiment control module 171 includes a resettable and programmable dwell timer implemented in software, hardware, or firmware and configurable via control panel 172. (In some embodiments, processor 171 takes the form of a programmable logic controller.) The sensor units include ozone, temperature, humidity, and/or pressure sensors that are hardwired or wirelessly linked to controller 171. Additionally, the sensor network includes shredder load sensors, for example in the form of a self-calibrating current sensing switches coupled to sense electrical current drawn by the shredder.

In some embodiments, ozone generator 161, which includes a humidifier for humidifying ozone gas. In some other embodiments, a humidity generatior module is used to control humidity with the treatment chamber. The humidity generator can take the form of a two-pressure two-temperature, hybrid humidity generator which generates humidity by fully saturating gas at a known temperature and pressure, then reducing the pressure to a lower value (typically ambient) and cooling or warming to an alternate temperature. Humidity produced by this hybrid technique is determined from measurements of the temperatures and pressures alone, and does not rely on measurement of the water vapor content of the gas. A generator of this type is used to establish relative humidity in excess of 70% within the chamber, and preferably grater than 80, 85, 90, and 95%. Moisture-saturated ozone gas has much higher oxidation potential than dry gas, enhancing sterilization efficacy of the ozone and reducing flammability.

In general operation, once controller 170 establishes proper sterilization conditions within chamber 140, waste material 101 is fed through hopper 120 onto shredder 130, where it is shredded and screened, falling under its own weight into the chamber 140. Auger 142 within the chamber then transport the shredded wait ad t controlled pace (for a defined period of time, such as 45, 50, 55, or 60 minutes depending on the ozone concentration and relative humidity) through the chamber, allowing it to move be fully exposed and sterilized when it reaches partition 143. AS the sterilized waste is pushed through port 143A, it backs up against exit member, sealing the chamber. As more waste is sterilized and feed into the exit portion of chamber, it eventually creates forces open the exit member, dropping into waste container 150. Notably, the shredder and auger operate continuously, that is without stoppage, as long as waste is available in the hopper and the shredder is shredding, providing increased throughput over typical systems that stop shredding to allow for static dwell of small batches of waste. In the exemplary embodiment, the dwell or treatment time is controlled by the control module, which includes a treatment bin timer, or dwell timer. The timer is set and selectively reset by the controller based on activity of the auger or shredder in some embodiments, specifically the electrical current load on the auger or shredder as sensed by sensor 159. More specifically, the exemplary system starts the timer with the initial start of the auger and every time the auger is stopped and restarted in sync with the shredder, ensuring that any waste that enters the chamber does not exit the chamber until it has gone through at least one full treatment period.

### Exemplary Method(s) of Operation of Exemplary System.

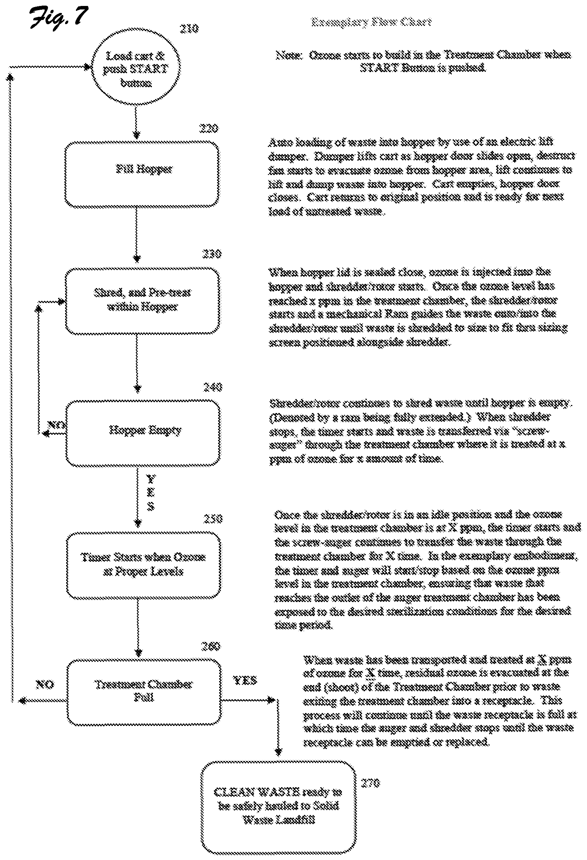

Fig. 7 shows a flow chart 700 of an exemplary method of operating a biomedical waste treatment system, such as system 100 or system 200. Flow chart 700 includes process blocks 710-790.
- **700** Prior to waste being lifted into hopper, ozone starts to build in the Treatment Chamber to the pre- programmed settings, such as preprogrammed Ozone ppm level, temperature, and/or humidity, stored in memory accessible via controller/processor. Some embodiments include multiple temperature, humidity, and ozone ppm levels, one set of levels or thresholds for various sections of the auger treatment chamber, allowing further control, monitoring, and/or regulation of sanitization conditions within the chamber.
- **710** The waste cart of any size e.g. 227, 360, 568 Liters (60, 95, 150 gal) etc. is placed on the electric lift dumper with PLC "green light indicator" start button, signaling operator that all parameters are ready for system operation.
- **720** The untreated waste receptacle can be weighed and recorded on the PLC (programmable logic controller) memory)prior to the electric lift dumper lifting. Once the lifting of the receptacle begins, the hopper door slides open, the ozone destruct fan starts to evacuate ozone from the hopper area, and lift continues to lift and dump waste into the hopper. The receptacle is then emptied and after X amount of seconds, for example, 10, 20, 30, 40, 50, 60, 90, 120, the receptacle returns to its original position and is ready for the next load of untreated waste.
- **730** Next the hopper lid is closed to seal off escape of ozone, and ozone is injected into the hopper area. When ozone is at a desired X ppm in the treatment Chamber, the screw auger and the shredder/rotor start operating. A mechanical Ram guides the waste onto/into the shredder/rotor until waste is shredded to a size allowing it to fall thru the sizing screen positioned alongside shredder/rotor and falls onto/into the (beginning point) of the screw ager.
- **740** The shredder/rotor continues to shred waste until hopper is empty and the mechanical ram stops. The waste is then transferred thru the treatment chamber via the screw ager where it is fully treated with X ppm of Ozone for X amount of time. **The Treatment Chamber, which houses the screw auger, contains X amount of ozone/water nozzles (Humidizone^{™}) injecting Humidizone^{™} into the treatment chamber to allow for an even and thorough distribution of Humidizone^{™} which treats the waste in the Treatment Chamber while the auger mixes and transfers the waste to the solid waste disposal receptacle.**
- **750** Once the shredder/rotor is in an idle position, the auger is programmed such that the untreated waste at the start of the auger will remain in the Treatment Chamber for approximately X amount of time exposed to Humidizone^{™} before reaching the exit of the Treatment Chamber. Prior to exiting the Treatment Chamber, the waste is fully treated/sterilized.
- **760** As the waste is ready to fall thru a (shoot) into a receptacle after treatment, the residual ozone from the exit point of treatment chamber is safely evacuated thru an ozone destruct system.
- **770** When the ozone parameters have been fully met and the timer for the load being treated expires, the receptacle can be safely handled and brought to a landfill.

### Conclusion

In the foregoing specification, specific embodiments have been described. However, one of ordinary skill in the art appreciates that various modifications and changes can be made without departing from the scope of the invention as set forth in the claims below. Accordingly, the specification and figures are to be regarded as illustrative rather than restrictive.

The benefits, advantages, solutions to problems, and any element(s) that may cause any benefit, advantage, or solution to occur or become more pronounced are not to be construed as critical, required, or essential features or elements of any or all the claims. The invention is defined solely by the appended claims

It will be appreciated that some embodiments may comprise one or more generic or specialized processors (or "processing devices") such as microprocessors, digital signal processors, customized processors and field programmable gate arrays (FPGAs) and unique stored program instructions (including both software and firmware) that control the one or more processors to implement, in conjunction with certain non-processor circuits, some, most, or all of the functions of the method and/or apparatus described herein. Alternatively, some or all functions could be implemented by a state machine that has no stored program instructions, or in one or more application specific integrated circuits (ASICs), in which each function or some combinations of certain of the functions are implemented as custom logic. Of course, a combination of the two approaches could be used.

Moreover, some embodiments or portions thereof can be implemented as a computer-readable volatile and/or non-volatile storage medium or memory (more generally a non-transitory machine-readable storage medium) having computer readable code or instructions stored thereon for directing operation of or causing programming of a computer (e.g., including one or more processors) to perform one or more portions of method as described and/or claimed herein. Exemplary computer-readable storage mediums include a hard disk, a CD-ROM, an optical storage device, a magnetic storage device, a ROM (Read Only Memory), a PROM (Programmable Read Only Memory), an EPROM (Erasable Programmable Read Only Memory), an EEPROM (Electrically Erasable Programmable Read Only Memory) and a Flash memory.

## Claims

1. A biomedical waste treatment assembly (100, 200) comprising:
a shredder (130); a hopper (120);
an ozone generator (160);
a longitudinal waste treatment chamber (140)_arranged to receive shredded biomedical waste from the shredder(130), the chamber (140) including:
a set of one or more ozone injection points (163C, 163D, 163E) coupled to the ozone generator (160) and_distributed along a length dimension of the chamber (140);
an auger (142B)_arranged to convey shredded waste along the length dimension of the chamber, the chamber configured to extend from a first height to a second height higher than the first height; and
a partition (143) dividing the chamber (140) into a treatment portion (141A) and an exit portion (141B), with the partition (143) being transverse to the length dimension of the chamber and having at least one opening (143A) for allowing treated waste to move from the treatment portion (141A) to the exit portion (141B), the opening having a cross-sectional area that is substantially less than that of the chamber.

2. The biomedical waste treatment assembly (100, 200) of claim 1 further comprising a processing circuit coupled to a memory, with the memory including instructions for starting and stopping the auger from moving shredded waste along the length dimension when desired ozone sterilization conditions are not satisfied.

3. The biomedical waste treatment assembly (100, 200) of claim 2 further comprising a timer associated with the processing circuit (160), the timer configured to measure a cumulative treatment time that shredded biomedical waste within the chamber has been subjected to the desired ozone sterilization conditions, wherein the timer is configured to be started and stopped by the processing circuit in sync with the auger being stopped and started.

4. The biomedical waste treatment assembly (100, 200) of claim 4 wherein the processing circuit (160) is further configured to start the auger (142) and the timer in response to the desired ozone sterilization conditions being established in the chamber (140) and to stop the auger (142) and the timer in response to the desired ozone sterilization conditions being determined to not be established, and to restart the timer in response to the desired conditions being re-established.

5. The biomedical waste treatment assembly (100, 200) of claim 2, 3, or 4 wherein the conditions include a desired ozone level and a desired humidity level within the chamber (140).

6. The biomedical waste treatment assembly (100, 200) of claim 5 wherein the conditions further include a desired temperature.

7. The biomedical waste treatment assembly (100, 200) of claim 5 wherein the conditions include a minimum desired ozone level as measured at a plurality of points along the length dimension of the chamber.

8. The biomedical waste treatment assembly (100, 200) of any of the claims above, further including means for establishing and maintaining relative humidity within chamber in excess of 70%.

9. The biomedical waste treatment assembly (100, 200) of any of the claims above, wherein the cross-sectional area of the opening (143A) in the partition (143) is at least 50% less than that of the chamber (140).

10. The biomedical waste treatment assembly (100, 200) of any of the claims above, further comprising:
means (144) for biasing treated waste shredded waste within the exit portion (141B) of the chamber (140) toward the opening (143A) in the partition (143) to facilitate maintaining pressure and ozone concentration within the treatment portion (141A) as waste is conveyed into the exit portion (141B) of the chamber (140).

11. The biomedical waste assembly (100, 200) of claim 10 wherein the means (144) for biasing includes a weighted door (144) that pivots open in response to shredded waste moved by the auger (142) through the opening (143A) n the partition (143) and against the door (144)

12. The biomedical waste assembly (100, 200) of claim 11 wherein the means (144) for biasing includes an automatic tensioning or resistance mechanism on the door (144) to dynamically adjust density of a plug seal of treated shredded waste against the opening (143A) to help maintain predetermined pressure conditions within the treatment portion (141A) of the chamber (140) or ozone levels within the exit portion (141B) of the chamber (140).

## Patentansprüche

1. Behandlungsanordnung biomedizinischer Abfälle (100, 200), umfassend:
einen Zerkleinerer (130); einen Trichter (120);
einen Ozongenerator (160);
eine längliche Abfallbehandlungskammer (140),_die angeordnet ist, um zerkleinerten biomedizinischen Abfall aus dem Zerkleinerer (130) zu erhalten, wobei die Kammer (140) Folgendes beinhaltet:
einen Satz von einem oder mehreren Ozoneinspritzpunkten (163C, 163D, 163E), die mit dem Ozongenerator (160) gekoppelt und entlang einer Längsabmessung der Kammer (140) verteilt sind;
eine Schnecke (142B)_die angeordnet ist, um zerkleinerten Abfall entlang der Längsabmessung der Kammer zu befördern, wobei die Kammer konfiguriert ist, um sich von einer ersten Höhe zu einer zweiten Höhe zu erstrecken, die höher ist als die erste Höhe; und
eine Trennwand (143), die die Kammer (140) in einen Behandlungsabschnitt (141 A) und einen Ausgangsabschnitt (141B) unterteilt, wobei die Trennwand (143) quer zu der Längsabmessung der Kammer ist und mindestens eine Öffnung (143A) aufweist, die es ermöglicht, dass behandelter Abfall von dem Behandlungsabschnitt (141A) zu dem Ausgangsabschnitt (141B) bewegt wird, wobei die Öffnung einen Querschnittsbereich aufweist, der im Wesentlichen kleiner ist als der der Kammer.

2. Behandlungsanordnung biomedizinischer Abfälle (100, 200) nach Anspruch 1, ferner umfassend eine Verarbeitungsschaltung, die mit einem Speicher gekoppelt ist, wobei der Speicher Anweisungen zum Starten und Anhalten der Schnecke zum Bewegen von zerkleinertem Abfall entlang der Längenabmessung beinhaltet, wenn gewünschte Ozonsterilisationsbedingungen nicht erfüllt sind.

3. Behandlungsanordnung biomedizinischer Abfälle (100, 200) nach Anspruch 2, ferner umfassend einen Zeitgeber, der mit der Verarbeitungsschaltung (160) assoziiert ist, wobei der Zeitgeber konfiguriert ist, um eine kumulative Behandlungszeit zu messen, in der zerkleinerter biomedizinischer Abfall in der Kammer den gewünschten Ozonsterilisationsbedingungen unterzogen wurde, wobei der Zeitgeber konfiguriert ist, um durch die Verarbeitungsschaltung synchron mit dem Anhalten und Starten der Schnecke gestartet und angehalten zu werden.

4. Behandlungsanordnung biomedizinischer Abfälle (100, 200) nach Anspruch 4, wobei die Verarbeitungsschaltung (160) ferner konfiguriert ist, um die Schnecke (142) und den Zeitgeber als Reaktion darauf zu starten, dass die gewünschten Ozonsterilisationsbedingungen in der Kammer (140) hergestellt sind, und um die Schnecke (142) und den Zeitgeber als Reaktion darauf anzuhalten, dass bestimmt wird, dass die gewünschten Ozonsterilisationsbedingungen nicht hergestellt sind, und um den Zeitgeber als Reaktion darauf neu zu starten, dass die gewünschten Bedingungen wieder hergestellt sind.

5. Behandlungsanordnung biomedizinischer Abfälle (100, 200) nach Anspruch 2, 3 oder 4, wobei die Bedingungen ein gewünschtes Ozonniveau und ein gewünschtes Feuchtigkeitsniveau innerhalb der Kammer (140) beinhalten.

6. Behandlungsanordnung biomedizinischer Abfälle (100, 200) nach Anspruch 5, wobei die Bedingungen ferner eine gewünschte Temperatur beinhalten.

7. Behandlungsanordnung biomedizinischer Abfälle (100, 200) nach Anspruch 5, wobei die Bedingungen ein minimales gewünschtes Ozonniveau beinhalten, das an einer Vielzahl von Punkten entlang der Längsabmessung der Kammer gemessen wird.

8. Behandlungsanordnung biomedizinischer Abfälle (100, 200) nach einem der obigen Ansprüche, die ferner eine Einrichtung zum Herstellen und Aufrechterhalten von relativer Feuchtigkeit innerhalb der Kammer von mehr als 70 % beinhaltet.

9. Behandlungsanordnung biomedizinischer Abfälle (100, 200) nach einem der obigen Ansprüche, wobei der Querschnittsbereich der Öffnung (143A) in der Trennwand (143) mindestens 50 % kleiner ist als der der Kammer (140).

10. Behandlungsanordnung biomedizinischer Abfälle (100, 200) nach einem der obigen Ansprüche, ferner umfassend:
Einrichtung (144) zum Ausrichten von behandeltem Abfall, zerkleinertem Abfall innerhalb des Ausgangsabschnitts (141B) der Kammer (140) in Richtung der Öffnung (143A) in der Trennwand (143), um ein Aufrechterhalten von Druck und Ozonkonzentration innerhalb des Behandlungsabschnitts (141A) zu erleichtern, wenn Abfall in den Ausgangsabschnitt (141B) der Kammer (140) befördert wird.

11. Behandlungsanordnung biomedizinischer Abfälle (100, 200) nach Anspruch 10, wobei die Einrichtung (144) zum Ausrichten eine beschwerte Tür (144) beinhaltet, die als Reaktion auf zerkleinerten Abfall, der von der Schnecke (142) durch die Öffnung (143A) n der Trennwand (143) und gegen die Tür (144) bewegt wird, aufgeschwenkt wird.

12. Behandlungsanordnung biomedizinischer Abfälle (100, 200) nach Anspruch 11, wobei die Einrichtung (144) zum Ausrichten einen automatischen Spann- oder Widerstandsmechanismus an der Tür (144) beinhaltet, um eine Dichte einer Pfropfenabdichtung aus behandeltem zerkleinertem Abfall gegen die Öffnung (143A) dynamisch einzustellen, um dazu beizutragen, vorbestimmte Druckbedingungen innerhalb des Behandlungsabschnitts (141A) der Kammer (140) oder Ozonniveaus innerhalb des Ausgangsabschnitts (141B) der Kammer (140) aufrechtzuerhalten.

## Revendications

1. Ensemble de traitement de déchets biomédicaux (100, 200) comprenant :
un broyeur (130) ; une trémie (120) ;
un générateur d'ozone (160) ;
une chambre longitudinale de traitement de déchets (140)_disposée de façon à recevoir les déchets biomédicaux broyés provenant du broyeur (130), la chambre (140) comprenant :
un ensemble d'un ou plusieurs points d'injection d'ozone (163C, 163D, 163E) couplés au générateur d'ozone (160) et_répartis le long d'une dimension longitudinale de la chambre (140) ;
une vis sans fin (142B)_disposée de façon à transporter les déchets broyés le long de la dimension longitudinale de la chambre, la chambre étant conçue pour s'étendre d'une première hauteur à une seconde hauteur supérieure à la première hauteur ; et
une cloison (143) divisant la chambre (140) en une partie de traitement (141A) et une partie de sortie (141B), la cloison (143) étant transversale à la dimension longitudinale de la chambre et présentant au moins une ouverture (143A) pour permettre le déplacement des déchets traités de la partie de traitement (141A) à la partie de sortie (141B), l'ouverture présentant une section transversale qui est sensiblement inférieure à celle de la chambre.

2. Ensemble de traitement de déchets biomédicaux (100, 200) selon la revendication 1, comprenant en outre un circuit de traitement couplé à une mémoire, la mémoire comprenant des instructions de démarrage et d'arrêt de la vis sans fin pour déplacer les déchets broyés le long de la dimension longitudinale lorsque les conditions de stérilisation à l'ozone souhaitées ne sont pas satisfaites.

3. Ensemble de traitement de déchets biomédicaux (100, 200) selon la revendication 2, comprenant en outre un temporisateur associé au circuit de traitement (160), le temporisateur étant conçu pour mesurer un temps de traitement cumulé pendant lequel les déchets biomédicaux broyés à l'intérieur de la chambre ont été soumis aux conditions de stérilisation à l'ozone souhaitées, ledit temporisateur étant conçu pour être démarré et arrêté par le circuit de traitement en synchronisation avec l'arrêt et le démarrage de la vis sans fin.

4. Ensemble de traitement de déchets biomédicaux (100, 200) selon la revendication 4, ledit circuit de traitement (160) étant en outre conçu pour démarrer la vis sans fin (142) et le temporisateur en réponse aux conditions de stérilisation à l'ozone souhaitées qui sont établies dans la chambre (140) et pour arrêter la vis sans fin (142) et le temporisateur en réponse au fait que les conditions de stérilisation à l'ozone souhaitées sont déterminées comme n'étant pas établies, et pour redémarrer le temporisateur en réponse au rétablissement des conditions souhaitées.

5. Ensemble de traitement de déchets biomédicaux (100, 200) selon la revendication 2, 3 ou 4, lesdites conditions comprenant le taux d'ozone souhaité et le taux d'humidité souhaité à l'intérieur de la chambre (140).

6. Ensemble de traitement de déchets biomédicaux (100, 200) selon la revendication 5, lesdites conditions comprenant en outre la température souhaitée.

7. Ensemble de traitement de déchets biomédicaux (100, 200) selon la revendication 5, lesdites conditions comprenant le taux d'ozone minimal souhaité tel que mesuré au niveau d'une pluralité de points le long de la dimension longitudinale de la chambre.

8. Ensemble de traitement de déchets biomédicaux (100, 200) selon l'une quelconque des revendications précédentes, comprenant en outre un moyen pour établir et maintenir l'humidité relative à l'intérieur de la chambre supérieure à 70 %.

9. Ensemble de traitement de déchets biomédicaux (100, 200) selon l'une quelconque des revendications précédentes, la section transversale de l'ouverture (143A) dans la cloison (143) étant au moins 50 % inférieure à celle de la chambre (140).

10. Ensemble de traitement de déchets biomédicaux (100, 200) selon l'une quelconque des revendications précédentes, comprenant en outre :
un moyen (144) pour solliciter les déchets broyés les déchets traités à l'intérieur de la partie de sortie (141B) de la chambre (140) en direction de l'ouverture (143A) dans la cloison (143) pour faciliter le maintien de la pression et de la concentration d'ozone à l'intérieur de la partie de traitement (141A) pendant que les déchets sont transportés dans la partie de sortie (141B) de la chambre (140).

11. Ensemble de déchets biomédicaux (100, 200) selon la revendication 10, ledit moyen (144) pour solliciter comprenant une porte lestée (144) qui pivote pour s'ouvrir en réponse aux déchets broyés déplacés par la vis sans fin (142) à travers l'ouverture (143A) n la cloison (143) et contre la porte (144).

12. Ensemble de déchets biomédicaux (100, 200) selon la revendication 11, ledit moyen (144) pour solliciter comprenant un mécanisme de mise en tension ou de résistance automatique sur la porte (144) pour ajuster dynamiquement la densité d'un joint de bouchon de déchets broyés traités contre l'ouverture (143A) pour aider à maintenir des conditions de pression prédéfinies à l'intérieur de la partie de traitement (141A) de la chambre (140) ou des taux d'ozone à l'intérieur de la partie de sortie (141B) de la chambre (140).
